# EUROPÄISCHE PATENTANMELDUNG

(11) **EP 2 664 699 A1**
(43) Veröffentlichungstag der Anmeldung: **20.11.2013**
(21) Anmeldenummer: 13167162.0
(22) Anmeldetag: 09.05.2013
(51) Int. Cl.: D02G 3/32

(54) **Elastischer Strickfaden sowie Verfahren zu seiner Herstellung**

(30) Priorität: 15.05.2012 DE 102012009582
(71) Anmelder: medi GmbH & Co. KG, 95448 Bayreuth (DE)
(72) Erfinder: Atmanspacher, Jan, 95485 Warmensteinach (DE)
(74) Vertreter: Blaumeier, Jörg

(57) **Zusammenfassung**

Elastischer Strickfaden (1), mit einer Seele (2) aus einem elastischen Material, einer die Seele umfassenden inneren Umwindung (3) und einer diese umfassenden äußeren Umwindung (4), wobei das eine Garn ein glattes Garn (5) und das andere Garn ein texturiertes Garn (6) ist.

## Beschreibung

Die Erfindung betrifft einen elastischen Strickfaden, mit einer Seele aus einem elastischen Material, einer die Seele umfassenden inneren Umwindung und einer diese umfassenden äußeren Umwindung.

Solche elastischen Strickfäden werden zur Herstellung unterschiedlichster Textilien verwendet, ein prominentes Beispiel ist die Herstellung von Strümpfen, primär Damenstrümpfen respektive Strumpfhosen, wie auch insbesondere in Form von Kompressionsstrümpfen, die zur Aufbringung respektive Erzeugen eines definierten Kompressionsdrucks, der aus medizinischen respektive therapeutischen Gesichtspunkten am Bein zu erzeugen ist, dienen. Ein bekannter Strickfaden umfasst eine Seele aus einem elastischen Material, welche aus einer inneren Umwindung aus einem ersten Garn umfasst ist, und um welche innere Umwindung eine äußere Umwindung aus einem zweiten Garn gedreht ist. Ein solches Garn ist beispielsweise aus DE 20 2010 013 665 U1 bekannt. Die beiden dort verwendeten Garne sind vom Typ her unterschiedlich. Die innere Umwindung ist mittels eines Microfasergarns gebildet, während die äußere Umwindung mit einem Feinstgarn oder Multifilamentgarn gefertigt sein kann. Die beiden verwendeten Garne gemäß DE 20 2010 013 665 U1 zeichnen sich durch eine besondere feuchte transportierende Eigenschaft aus, nachdem der beschriebene Faden respektive ein hieraus hergestellter medizinischer Kompressionsstrumpf einen besonders guten Feuchtigkeitstransport gewährleisten soll.

Bei aus einem Strickfaden der eingangs genannten Art hergestellten Kompressionsgestricken besteht jedoch mitunter das Problem, dass das Gestrick, also beispielsweise der Kompressionsstrumpf, aufgrund der sehr hohen Rücksprungkraft des Strickfadens häufig schwer anziehbar ist, denn aufgrund dieser hohen Rücksprungkraft dehnt sich das Gestrick beim Anziehen stark, was das Anziehen erschwert.

Der Erfindung liegt damit das Problem zugrunde, einen elastischen Strickfaden anzugeben, der ein verbessertes Rücksprungverhalten aufweist, sodass daraus gefertigte Textilien wie insbesondere Kompressionsstrümpfe einfacher anziehbar sind.

Zur Lösung dieses Problems ist bei einem elastischen Strickfaden der eingangs genannten Art erfindungsgemäß vorgesehen, dass das eine Garn ein glattes Garn und das andere Garn ein texturiertes Garn ist.

Erfindungsgemäß werden also zwei unterschiedliche Garne verwendet, von denen eines ein glattes Garn ist, während das andere ein definiert im Herstellprozess respektive in der Garnaufbereitung texturiertes Garn ist. Ein solches Garn wird im Herstellprozess definiert verformt, sodass es sich leicht kräuselt. Werden nun ein glattes Garn und ein texturiertes Garn zur Bildung der beiden Umwindungen verwendet, so ergibt sich ein unterschiedliches Dehnverhalten über die Fadenlänge. Während das texturierte Garn aufgrund seiner Texturierung, also Kräuselung, relativ stark gedehnt werden kann, ist der glatte Faden deutlich geringer dehnbar. Hieraus resultiert folglich, dass sich der gesamte hybride Aufbau, also der Strickfaden, in einem deutlich geringerem Maß dehnt, verglichen mit einem elastischen Strickfaden, der aus zwei texturierten Garnen gefertigt ist. Aufgrund des reduzierten Rücksprungverhaltens ergibt sich folglich ein deutlich anderes Dehnverhalten des fertigen Kompressionsgestricks, also des Kompressionsstrumpfes oder dergleichen, sodass es, wie sich überraschend herausgestellt hat, deutlich einfacher und angenehmer angezogen werden kann.

Bevorzugt bildet das glatte Garn die innere Umwindung, während das texturierte Garn die äußere Umwindung bildet. Die Bildung der äußeren Umwindung mittels des texturierten Garnes verleiht dem Gestrick eine sehr angenehme Haptik, die für ein angenehmes Tragegefühl vorteilhaft ist.

Bevorzugt wird als texturiertes Garn ein vorgeschrumpftes Garn verwendet. Dies ist dahingehend vom Vorteil, als im Rahmen eines Färbeprozesses des fertigen Gestricks, wenn diesem also eine bestimmte Farbe verliehen werden soll, was über einen thermischen Prozess erfolgt, das texturierte Garn nicht oder nur geringfügig schrumpft, während das glatte Garn demgegenüber etwas stärker schrumpft. Hierüber kann sichergestellt werden, dass es während des Färbeprozesses nicht zu größeren, gegebenenfalls unkalkulierbaren Eigenschaftsänderungen hinsichtlich der Gestrickdehnung kommt. Selbstverständlich kann auch das glatte Garn vorgeschrumpft sein.

Als Garne können Microfasergarne, Multifilamentgarne oder normalkapillare Garne verwendet werden, wobei die beiden Garne gleich oder unterschiedlich sein können. D. h., dass die innere Umwindung beispielsweise aus einem Microfasergarn und die äußere Umwindung um einen Multifilamentgarn bestehen kann, wenngleich selbstverständlich auch gleichartige Garne wie beispielsweise zwei Multifilamentgarne zur Bildung der inneren und äußeren Umwindung verwendet werden können.

Gemäß einer besonders zweckmäßige Weiterbildung der Erfindung ist vorgesehen, dass die beiden Garne mit unterschiedlicher Tourenzahl um die Seele gedreht sind. D. h., dass entweder das erste oder das zweite Garn mit mehr Touren als das jeweils andere Garn gedreht ist. Hierüber kann eine Einstellung des Dehnverhaltens erreicht werden. Je nachdem, ob nun das glatte oder das texturierte Garn mit höherer Tourenzahl gedreht ist, variiert der jeweilige Garnanteil pro Fadenmeter, woraus sich, nachdem die beiden Garne ein unterschiedliches Dehnverhalten besitzen, das Gesamtdehnverhalten entsprechend verändert. Selbstverständlich besteht grundsätzlich die Möglichkeit, die beiden Garne auch mit gleicher Tourenzahl zu drehen.

Bevorzug ist dabei das texturierte Garn, insbesondere wenn dieses die äußere Umwindung bildet, mit einer höheren Tourenzahl gedreht.

Die Tourenzahl des jeweiligen Garns selbst sollte zwischen 500 - 2000 betragen. Sie liegt vorzugsweise im Bereich zwischen 600 - 1500 Touren, insbesondere zwischen 700 - 1200 Touren und besonders bevorzugt zwischen 800 - 1000 Touren. Sind die beiden Garne mit unterschiedlicher Tourenzahl gedreht, so sollte die Tourendifferenz wenigstens 25 Touren betragen, vorzugsweise wenigstens 50. Die Differenz kann auch mehr Touren betragen, beispielsweise 100 oder 150 Touren, wobei sich auch hierüber selbstverständlich das Dehnverhalten des Fadens variieren lässt.

Die Seele selbst besteht vorzugsweise aus Elastan, während die Garne aus PA6 oder PA6.6 bestehen. Selbstverständlich sind auch andere Materialien zur Bildung der Seele respektive Garne verwendbar.

Neben dem Faden selbst betrifft die Erfindung ein textiles Gestrick, hergestellt aus einem elastischen Strickfaden der beschriebenen Art. Das Gestrick ist bevorzugt ein Kompressionsstrumpf.

Weiterhin betrifft die Erfindung ein Verfahren zur Herstellung eines elastischen Strickfadens der beschriebenen Art, das sich dadurch auszeichnet, dass um eine Seele aus elastischem Material eine innere Umwindung aus einem ersten Garn und um diese Umwindung eine äußere Umwindung aus einem zweiten Garn gedreht wird, wobei ein Garn ein glattes Garn und das andere Garn ein texturiertes Garn ist.

Dabei kann zur Bildung der inneren Umwindung bevorzugt das glatte Garn verwendet werden, während das texturierte Garn zur Bildung der äußeren Umwindung verwendet wird. Dies ist insbesondere aus Gründen der Haptik zweckmäßig. Einer umgekehrten Anordnung steht jedoch jedenfalls nichts entgegen.

Als texturiertes Garn wird vorzugsweise ein vorgeschrumpftes Garn verwendet, wobei natürlich auch das glatte Garn vorgeschrumpft sein kann.

Grundsätzlich können als Garne Microfasergarne, Multifilamentgarne oder normalkapillare Garne verwendet werden, wobei die beiden verwendeten Garne gleichartig oder unterschiedlich sein können. Bevorzugt jedoch werden gleichartige Garne in Form von Multifilamentgarnen verwendet. Die Differenzierung zwischen Microfasergarn, Multifilamentgarn und normalkapillaren Garn erfolgt über das Verhältnis aus Titer zu Fadenzahl. Der Titer gibt die Garnstärke an, seine Einheit wird in "dtex" angegeben, also decitex, wobei 1 dtex einem Fadengewicht von 1 g/10.000 m entspricht. Die Fadenanzahl ist ebenfalls variabel. Bei einem Verhältnis von Titer/Fadenzahl von ≤ 1 spricht man Microfasergarn, bei einem Verhältnis von > 1,0 - 2,5 von einem Multifilamentgarn, und einem Verhältnis > 2,5 von einem normalkapillaren Garn.

In Weiterbildung der Erfindung zeichnet sich das Verfahren dadurch aus, dass die beiden Garne vorzugsweise mit unterschiedlicher Tourenzahl um die Seele gedreht werden, wobei die jeweilige Tourenzahl zwischen 500 - 2000 Touren, vorzugsweise 600 - 1500 Touren, insbesondere zwischen 700 - 1200 Touren und besonders bevorzugt zwischen 800 - 1000 Touren beträgt. Jedweder Zwischenwert gilt als erfindungswesentlich offenbart. Natürlich können beide Garne auch mit gleicher Tourenzahl gedreht sein.

Bei Drehung mit unterschiedlichen Touren sollte die Tourendifferenz wenigstens 25 Touren betragen, kann aber auch größer sein. Bevorzugt wird dabei das texturierte Garn mit einer höheren Tourenzahl gedreht, insbesondere wenn es das die äußere Umwindung bildet.

Als Materialien wird bevorzugt eine Seele aus Elastan verwendet, als Garnmaterialien werden PA6 oder PA6.6 verwendet.

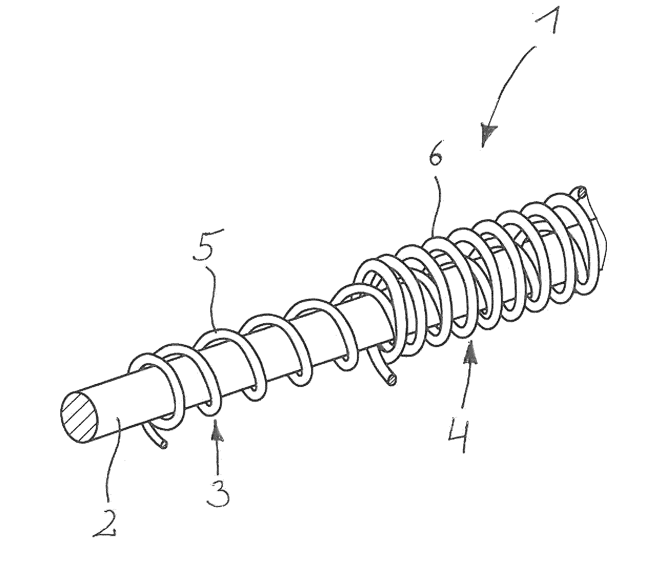

Weitere Vorteile, Merkmale und Einzelheiten der Erfindung ergeben sich aus dem im Folgenden beschriebenen Ausführungsbeispiel sowie anhand der Zeichnung.

In dieser Prinzipdarstellung ist eine erfindungsgemäßer elastischer Strickfaden 1 gezeigt, bestehend aus einer Seele 2 aus vorzugsweise Elastan, einer inneren Umwindung 3, die unmittelbar um die Seele 2 gedreht ist, sowie einer äußeren Umwindung 4, die die innere Umwindung 3 umgibt. Die innere Umwindung 3 ist im gezeigten Ausführungsbeispiel aus einem glatten Garn 5 gedreht, während die äußere Umwindung 4 aus einem texturierten Garn 6 gedreht ist. Die Garne bestehen bevorzugt aus PA6 oder PA6.6. Es handelt sich bei den Garnen 5, 6 bevorzugt um zwei Multifilamentgarne, die sich jedoch wie ausgeführt dadurch unterscheiden, dass das Garn 5 glatt ist, während das Garn 6 texturiert ist.

Ersichtlich ist das erste Garn 5 mit einer niedrigeren Tourenzahl um die Seele 2 gedreht, als das zweite Garn 6. Beispielsweise kann das erste Garn mit einer Tourenzahl von 850 und das zweite Garn 6 mit einer Tourenzahl von 900 Touren gedreht sein. Die Drehrichtung beider Garne 5, 6 ist im gezeigten Beispiel gleich, es ist eine S/S-Drehung. Denkbar sind aber natürlich auch andere Drehungen, wie eine Drehung beider Garne 5, 6 in die andere Richtung, also eine Z/Z-Drehung oder unterschiedliche Drehrichtungen, also S/Z- respektive Z/S-Drehungen.

Nachdem das texturierte Garn 6 hier die äußere Umwindung 4 bildet, ergibt sich im hieraus gefertigten Gestrick eine sehr angenehme Haptik, die für den Tragekomfort vorteilhaft ist.

Ferner weisen beide Garne 5, 6 ein unterschiedliches Dehnverhalten auf. Das bevorzugt vorgeschrumpfte texturierte Garn 6 kann deutlich stärker gedehnt werden, da es aufgrund seiner Texturierung weiter gelängt werden kann, als das glatte Garn 5. Hieraus ergibt sich eine reduzierte Rücksprungkraft des Fadens 1, d. h., dass der Faden insgesamt etwas weniger stark dehnbar ist, was sich vorteilhaft für das daraus hergestellte Gestrick auswirkt. Die Anziehbarkeit eines aus diesem Faden 1 hergestellten Strumpfs wird vereinfacht.

Ein bevorzugter Strickfaden, der sich zur Herstellung von Kompressionsstrümpfen besonders geeignet hat, weist eine Seele aus 44/45 dtex Elastan auf. Die innere Umwindung 3 ist mit einem PA6.6-Garn mit 33 dtex / 20 Fäden als glattes Garn ausgeführt, während die äußere Umwindung 4 mit einem PA6.6-Garn mit ebenfalls 33 dtex / 20 Fäden ausgeführt ist. Das innere Garn 5 ist mit 850 Touren gedreht, während das äußere Garn 6 mit 900 Touren gedreht ist, bei einer S/S-Drehung. Mithin ist eine Tourendifferenz von 50 gegeben. Dies ist jedoch lediglich ein Ausführungsbeispiel, die Erfindung ist hierauf nicht beschränkt. Selbstverständlich sind auch andere Garne 5, 6 mit anderen Dehnungsverhalten respektive Fadenanzahlen bei anderen Touren respektive Drehrichtung verwendbar.

## Patentansprüche

1. Elastischer Strickfaden, mit einer Seele aus einem elastischen Material, einer die Seele umfassenden inneren Umwindung und einer diese umfassenden äußeren Umwindung, **dadurch gekennzeichnet, dass** das eine Garn ein glattes Garn (5) und das andere Garn ein texturiertes Garn (6) ist.

2. Elastischer Strickfaden nach Anspruch 1, **dadurch gekennzeichnet, dass** das glatte Garn (5) die innere Umwindung (3) und das texturierte Garn (6) die äußere Umwindung (4) bildet, oder umgekehrt.

3. Elastischer Strickfaden nach Anspruch 1 oder 2, **dadurch gekennzeichnet, dass** das texturierte Garn (6) vorgeschrumpft ist.

4. Elastischer Strickfaden nach einem der vorangehenden Ansprüche, **dadurch gekennzeichnet, dass** die Garne (5, 6) Microfasergarne, Multifilamentgarne oder normalkapillare Garne sind, wobei die beiden Garne gleichartig oder unterschiedlich sind.

5. Elastischer Strickfaden nach Anspruch 4, **dadurch gekennzeichnet, dass** beide Garne (5, 6) Multifilamentgarne sind.

6. Elastischer Strickfaden nach einem der vorangehenden Ansprüche, **dadurch gekennzeichnet, dass** die beiden Garne (5, 6) mit gleicher oder unterschiedlicher Tourenzahl um die Seele (2) gedreht sind.

7. Elastischer Strickfaden nach Anspruch 6, **dadurch gekennzeichnet, dass** das texturierte Garn (6) mit einer höheren Tourenzahl gedreht ist.

8. Elastischer Strickfaden nach Anspruch 6 oder 7, **dadurch gekennzeichnet, dass** die Tourenzahl zwischen 500 - 2000 Touren, vorzugsweise zwischen 600 - 1500 Touren, insbesondere zwischen 700 - 1200 Touren und besonders bevorzugt zwischen 800 - 1000 Touren beträgt.

9. Elastischer Strickfaden nach einem der Ansprüche 6 bis 8, **dadurch gekennzeichnet, dass** bei unterschiedlicher Tourenzahn die Tourendifferenz wenigstens 25 Touren beträgt.

10. Elastischer Strickfaden nach einem der vorangehenden Ansprüche, **dadurch gekennzeichnet, dass** die Seele (2) aus Elastan und die Garne (5, 6) aus PA6 oder PA6.6 bestehen.

11. Elastischer Faden nach einem der vorangehenden Ansprüche, **dadurch gekennzeichnet, dass** die beiden Garne (5, 6) in S/S-, Z/Z-, S/Z- oder Z/S-Drehung gedreht sind.

12. Verfahren zur Herstellung eines elastischen Strickfadens nach einem der vorangehenden Ansprüche, **dadurch gekennzeichnet, dass** um eine Seele aus elastischem Material eine innere Umwindung aus einem ersten Garn und um diese Umwindung eine äußere Umwindung aus einem zweiten Garn gedreht wird, wobei ein Garn ein glattes Garn und das andere Garn ein texturiertes Garn ist.

13. Verfahren nach Anspruch 12, **dadurch gekennzeichnet, dass** zur Bildung der inneren Umwindung das glatte Garn und zur Bildung der äußeren Umwindung das texturierte Garn verwendet wird, oder umgekehrt.

14. Verfahren nach Anspruch 12 oder 13, **dadurch gekennzeichnet, dass** als texturiertes Garn ein vorgeschrumpftes Garn verwendet wird.

15. Verfahren nach einem der Ansprüche 12 bis 14, **dadurch gekennzeichnet, dass** als Garne Microfasergarne, Multifilamentgarne oder normalkapillare Garne verwendet werden, wobei die beiden verwendeten Garne gleichartig oder unterschiedlich sind, wobei insbesondere gleichartige Garne in Form von Multifilamentgarnen verwendet werden.

16. Verfahren nach einem der Ansprüche 12 bis 15, **dadurch gekennzeichnet, dass** die beiden Garne mit gleicher oder unterschiedlicher Tourenzahl um die Seele gedreht werden, wobei die Tourenzahl zwischen 500 - 2000 Touren, vorzugsweise zwischen 600 - 1500 Touren, insbesondere zwischen 700 - 1200 Touren und besonders bevorzugt zwischen 800 - 1000 Touren beträgt.

17. Verfahren nach Anspruch 16, **dadurch gekennzeichnet, dass** bei Drehung mit unterschiedlichen Touren die Tourendifferenz wenigstens 25 Touren beträgt.

18. Verfahren nach einem Anspruch 16 oder 17, **dadurch gekennzeichnet, dass** das texturierte Garn mit einer höheren Tourenzahl gedreht wird.

19. Verfahren nach einem der Ansprüche 12 bis 18, **dadurch gekennzeichnet, dass** eine Seele aus Elastan und Garne aus PA6 oder PA6.6 verwendet werden.

20. Textiles Gestrick, hergestellt aus einem elastischen Strickfaden nach einem der Ansprüche 1 bis 11.

21. Textiles Gestrick nach Anspruch 20, **dadurch gekennzeichnet, dass** es ein Kompressionsstrumpf ist.
